# EUROPEAN PATENT APPLICATION

(11) **EP 3 791 839 A1**
(43) Date of publication of application: **17.03.2021**
(21) Application number: 19800775.9
(22) Date of filing: 10.05.2019
(51) Int. Cl.: A61F 13/02, A61L 15/20, C09J 7/25, C09J 7/38, C09J 11/06, C09J 201/00

(54) **PATCH MATERIAL**

(30) Priority: 11.05.2018 JP 2018091878
(71) Applicant: NITTO DENKO CORPORATION, Ibaraki-shi Osaka 567-8680 (JP)
(72) Inventor: OZAWA Keita, Ibaraki-shi, Osaka 567-8680 (JP); YOSHIDA Noboru, Ibaraki-shi, Osaka 567-8680 (JP); YOSHIYAMA Makiko, Ibaraki-shi, Osaka 567-8680 (JP)
(74) Representative: Grünecker Patent- und Rechtsanwälte PartG mbB
(86) International application number: PCT/JP2019/018789
(87) International publication number: WO 2019/216423

(57) **Abstract**

The present invention relates to a patch including a polyurethane film, a first adhesive layer provided on one surface of the polyurethane film, and a cover tape provided on the other surface of the polyurethane film, wherein the cover tape includes a base material and a second adhesive layer provided on one surface of the base material and an adhesive surface of the second adhesive layer is laminated on the other surface-side of the polyurethane film, and the first adhesive layer contains an adhesive and an organic liquid component.

## Description

### TECHNICAL FIELD

The present invention relates to a patch. Particularly, the present invention relates to a skin patch for sticking on a skin surface.

### BACKGROUND ART

For the purpose of the protection of a wound on a skin and the prevention of contamination, it is general that the wound on the skin is covered with an adhesive sheet. Furthermore, in watching sports, events and the like, a skin of the face, the arm and the like is being decorated with the related design or colored pattern. A method of decoration by adhering an adhesive sheet having a design or a colored pattern previously printed thereon to a skin surface is proposed as the method of decorating a skin (for example, see Patent Literatures 1 to 3).

### CITATION LIST

### PATENT LITERATURE

Patent Literature 1: JP-A-H7-67912
Patent Literature 2: JP-A-2000-160111
Patent Literature 3: JP-A-2005-348974

### SUMMARY OF INVENTION

### TECHNICAL PROBLEM

As an adhesive sheet sticking to a skin surface, an adhesive layer having high flexibility so as to follow the movement of a skin surface is required in order to relieve discomfort during wear on a skin. However, the adhesive sheet having high flexibility has a problem of difficulty in handling such that wrinkles are easy to be generated in the adhesive sheet or adhesive surfaces of the adhesive sheet adhere to each other when sticking to a skin surface. For these reasons, the improvement is made that by the constitution that a protective film has been laminated on a surface opposite an adhesive layer-laminated surface of the adhesive sheet, stiffness is imparted to an adhesive sheet having high flexibility, thereby improving the handling properties of the adhesive sheet. However, the protective film laminated on the adhesive sheet is peelably laminated. Therefore, the protective film is easy to be peeled, and when the protective film is once peeled, the protective film is difficult to be again laminated on the adhesive sheet. Thus, further improvement is required.

In view of the above present situation, an object of the present invention is to provide a patch that is difficult to generate wrinkles when sticking to a skin surface and can be easily and beautifully stuck. Additionally, an object of the present invention is to provide a patch using a cover tape having an adhesive layer as a protective film, so that peeling of the cover tape is prevented during storage of the patch and even through the cover tape has been once peeled, the cover tape can be laminated again.

### SOLUTION TO PROBLEM

As a result of intensive investigations to achieve the above objects, the present inventors have found that the above problem can be solved, and completed the present invention.

Specifically, the present invention is described below.
(1) A patch including a polyurethane film, a first adhesive layer provided on one surface of the polyurethane film, and a cover tape provided on the other surface of the polyurethane film,
   wherein the cover tape includes a base material and a second adhesive layer provided on one surface of the base material and an adhesive surface of the second adhesive layer is laminated on the other surface-side of the polyurethane film, and
   the first adhesive layer contains an adhesive and an organic liquid component.
(2) The patch described in (1), wherein a printed layer is formed on at least a part of the other surface of the polyurethane film.
(3) The patch described in (1) or (2), wherein the content of the organic liquid component in the first adhesive layer is 10 to 60 mass %.
(4) The patch described in any one of (1) to (3), wherein the organic liquid component contains a fatty acid ester.
(5) The patch described in any one of (1) to (4), wherein the adhesive contains an acrylic adhesive.

### ADVANTAGEOUS EFFECTS OF INVENTION

The patch of the present invention can improve handleability when sticking to a skin surface by laminating the cover tape on the polyurethane film, thereby imparting stiffness to the patch. Furthermore, the cover tape is prevented from peeling during storing the patch, and additionally even though the cover tape is erroneously peeled, the cover tape can be again adhered to the polyurethane film and can protect the surface of the polyurethane film from scratches or the like. When the patch of the present invention has been adhered to a skin surface of the face, the arm and the like and then the cover tape has been removed, the patch has excellent stickiness to a skin surface. Additionally, the patch flexibly expands and contracts according to the movement of a skin surface, and has good fit feeling so as not feel stuck. Furthermore, after use, physical skin irritation when peeling the patch from a skin surface can be suppressed.

### BRIEF DESCRIPTION OF DRAWINGS

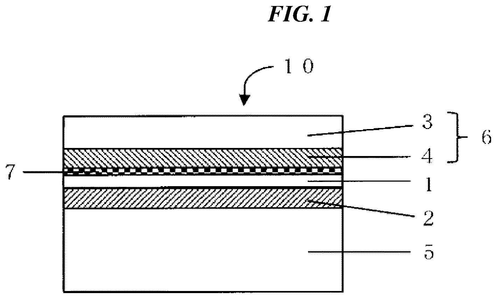

FIG. 1 is a schematic cross-sectional view showing one embodiment of the patch of the present invention. The Figure is drawn by enlarging in a vertical direction to the patch in order to intelligibly explain the concept of the present invention.

### DESCRIPTION OF EMBODIMENTS

The patch of the present invention is a patch including a polyurethane film, a first adhesive layer provided on one surface (hereinafter referred to as a "first surface") of the polyurethane film, and a cover tape provided on the other surface (hereinafter referred to as a "second surface") of the polyurethane film, the cover tape includes a base material and a second adhesive layer provided on one surface of the base material, an adhesive surface of the second adhesive layer is laminated on the other surface-side of the polyurethane film, and the first adhesive layer contains an adhesive and an organic liquid component. In other words, the patch is that the first adhesive layer is provided on one surface of the polyurethane film, the second adhesive layer-surface of the cover tape including the base material and the second adhesive layer is laminated on the other surface of the polyurethane film, and the first adhesive layer contains an adhesive and an organic liquid component.

The first surface and the second surface of the polyurethane film are two main surfaces facing to each other in the polyurethane film.

Urethane resin contained in the polyurethane film in the present invention is not particularly limited, and a general urethane resin such as an ether type urethane resin or an ester type urethane resin can be used.

The upper limit of a thickness of the polyurethane film is preferably 50 µm, more preferably 30 µm, still more preferably 20 µm, particularly preferably 15 µm and especially 9 µm, and the lower limit thereof is preferably 1 µm and more preferably 3 µm. When the thickness of the polyurethane film is less than 1 µm, the patch may break when using the patch or when peeling the patch from a skin surface after use. On the other hand, when the thickness of the polyurethane film exceeds 50 µm, flexibility of the patch becomes poor. As a result, while the patch sticks on the skin surface, uncomfortable feeling may occur to flexible movement of a skin surface, and stuffiness, a rash due to stuffiness, and the like may occur on a skin surface.

A printed layer is preferably formed on at least a part of the other surface (second surface) of the polyurethane film. The printed layer is formed by flexo printing, gravure printing, offset printing, inkjet printing, laser printer or the like that is generally performed. An ink used in the printed layer generally includes an ink comprising a varnish obtained by dissolving a pigment, oils and fats, a natural resin, a synthetic resin or the like in a solvent, as a main ingredient, and a slight amount of an additive such as a lubricant or a curing agent. Hue of the ink used in the printed layer can be appropriately determined depending on a use of the patch. For example, in the uses of watching sports, events and the like, the patch having hue of the related design and colored pattern is often used, and in the uses of wound dressing and the like, the patch having hue close to a skin color, that is unnoticeable when stuck to a skin, is often used.

The thickness of the printed layer is not particularly limited. However, to form a uniform printed layer without the occurrence of stuffiness and unevenness, the upper limit is preferably 10.0 µm and more preferably 5.0 µm, and the lower limit is preferably 0.1 µm and more preferably 0.5 µm.

In the present invention, the adhesive used in the first adhesive layer is not particularly limited, and adhesives generally used can be used. The adhesive includes an acrylic adhesive, a rubber adhesive, a silicone adhesive, a urethane adhesive, a vinyl ether adhesive, a vinyl ester adhesive and a polyester adhesive. Those adhesives may be used in one kind alone or as mixtures of two or more kinds. Above all, an acrylic adhesive is preferably used from easy adjustment of adhesive force and less skin irritation when being in contact with a skin surface.

The acrylic adhesive is not particularly limited, and, for example, an acrylic adhesive obtained from a monomer mixture containing a (meth)acrylic acid alkyl ester monomer is preferably used.

The (meth)acrylic acid alkyl ester monomer is a main component that imparts good stickiness to the adhesive layer, and in particular, use of an alkyl group having 6 or more carbon atoms and especially a long chain alkyl group having 6 to 18 carbon atoms is effective. The (meth)acrylic acid alkyl ester monomer has the advantages that irritation to a skin is relatively less and deterioration of adhesive force is difficult to occur even by the use for a long period of time.

Specific examples of the (meth)acrylic acid alkyl ester monomer include butyl ester, propyl ester, octyl ester, nonyl ester, decyl ester, dodecyl ester and lauryl ester of acrylic acid or methacrylic acid. Those can be used alone or as mixtures of two or more kinds. Those alkyl ester chains may be a straight chain and may be a branched chain.

As the acrylic adhesive, it is preferable to use an acrylic adhesive obtained from a monomer mixture containing an alkoxy group-containing ethylenically unsaturated monomer in order to impart water vapor permeability, a so-called moisture permeability. The alkoxy group-containing ethylenically unsaturated monomer is not particularly limited, but an alkoxyalkyl acrylate having an alkoxy group having 1 to 4 carbon atoms such as methoxypolyethylene glycol acrylate, ethoxydiethylene glycol acrylate, butoxydiethylene glycol acrylate, methoxyethyl acrylate, ethoxyethyl arylate or butoxyethyl acrylate is preferably used.

The acrylic adhesive may be an acrylic adhesive obtained from a monomer mixture further containing a carboxyl group-containing ethylenically unsaturated monomer. By containing the carboxyl group-containing ethylenically unsaturated monomer, cohesive force of the adhesive obtained is improved, thereby becoming a monomer important in adjusting properties of the adhesive layer. Representative examples of the monomer include acrylic acid, methacrylic acid, itaconic acid, crotonic acid, fumaric acid and (anhydrous) maleic acid. Of those, the monomer that is preferred in copolymerizability and handleability includes acrylic acid and methacrylic acid.

The acrylic adhesive contained in the first adhesive layer is preferably an acrylic adhesive obtained by copolymerization of a (meth)acrylic acid alkyl ester monomer and an alkoxy group-containing ethylenically unsaturated monomer and more preferably an acrylic adhesive obtained by copolymerization of a (meth)acrylic acid alkyl ester monomer, an alkoxy group-containing ethylenically unsaturated monomer and a carboxyl group-containing ethylenically unsaturated monomer.

In the acrylic adhesive, in addition to the above-mentioned respective monomers, a monomer such as styrene, vinyl acetate or N-vinyl-2-pyrrolidone may appropriately be copolymerized as a modifying monomer for performing various modifications such as impartation of hydrophilicity, as necessary.

The content of the (meth)acrylic acid alkyl ester monomer constituting the acrylic adhesive is that the upper limit is preferably 80 mass % and more preferably 75 mass % and the lower limit is preferably 40 mass % and more preferably 50 mass %. When the content of the (meth)acrylic acid alkyl ester monomer is in the above range, the acrylic adhesive shows good stickiness and additionally adhesive residue phenomenon does not occur when peeling from an adherend, thus showing excellent peelability.

The content of the alkoxy group-containing ethylenically unsaturated monomer in the acrylic adhesive is that the upper limit is preferably 60 mass % and more preferably 50 mass % and the lower limit is preferably 10 mass %, more preferably 20 mass % and still more preferably 25 mass %. When the content of the alkoxy group-containing ethylenically unsaturated monomer is in the above range, excellent moisture permeability can be imparted to the adhesive layer, and the effect that stuffiness and a rash due to stuffiness during sticking to a skin are reduced is exhibited.

In the case of containing the carboxyl group-containing ethylenically unsaturated monomer, the content of the alkoxy group-containing ethylenically unsaturated monomer is that the upper limit is preferably 50 mass % and more preferably 45 mass % and the lower limit is preferably 10 mass % and more preferably 20 mass %.

The content of the carboxyl group-containing ethylenically unsaturated monomer is that the upper limit is preferably 10 mass % and more preferably 8 mass % and the lower limit is preferably 1 mass % and more preferably 3 mass %. When the content of the carboxyl group-containing ethylenically unsaturated monomer is in the above range, excellent cohesive force can be imparted to the adhesive layer, and adhesive residue phenomenon and the like can be suppressed.

In the present invention, an organic liquid component is contained in the first adhesive layer. The organic liquid component imparts flexibility to the adhesive layer and additionally can reduce physical skin irritation when peeling the patch from a skin surface.

The organic liquid component is not particularly limited so long as it is a liquid state or a paste state at room temperature (25°C), or in the case that two or more kinds are mixed and used, a final mixture is a liquid state or a paste state at room temperature (25°C). The organic liquid component includes glycols such as ethylene glycol, propylene glycol, butylene glycol, diethylene glycol, dipropylene glycol, triethylene glycol, tripropylene glycol, polyethylene glycol and polypropylene glycol; vegetable oils and fats such as olive oil and castor oil; animal fats and oils such as liquid lanorin; hydrocarbons such as squalane and liquid paraffin; various surfactants; ethoxylated stearyl alcohol; long chain alcohols such as isostearyl alcohol and oleyl alcohol; higher fatty acids such as caprylic acid, capric acid, myristic acid, oleic acid and linoleic acid; pyrrolidones such as N-methylpyrrolidone and N-dodecylpyrrolidone; sulfoxides such as decylmethyl sulfoxide; esters between phthalic acid, maleic acid, adipic acid, stearic acid or various fatty acids and an alkyl alcohol; and esters with polyhydric alcohol, such as ethylene glycol and glycerin. Those can be used in one kind alone or as mixtures of two or more kinds.

Above all, esters between various fatty acids and an alkyl alcohol, and esters with polyhydric alcohol, such as ethylene glycol or glycerin, are preferred. More specifically, an ester of a monohydric alcohol includes dibutyl phthalate, di-2-ethylhexyl phthalate, dibutyl adipate, di-2-ethylhexyl sebacate, dibutyl maleate, ethyl myristate, isopropyl myristate, isopropyl palmitate, butyl stearate, isopropyl isostearate, hexyl laurate, cetyl lactate, myristyl lactate, diethyl phthalate, octyldodecyl myristate, octyldodecyl oleate, hexyldecyl dimethyl octanoate, cetyl 2-ethylhexanoate, isocetyl 2-ethylhexanoate, stearyl 2-ethylhexanoate and dioctyl succinate. An ester of a divalent or more alcohol includes propylene glycol dicaprylate, propylene glycol dicaprate, propylene glycol diisostearate, glyceryl monocaprylate, glyceryl tricaprylate, glyceryl tri-2-ethylhexanoate, glyceryl tricaplate, glyceryl trilaurate, glyceryl triisostearate, glyceryl trioleate and trimethylolpropane tri-2-ethylhexanoate. Those can be used in one kind or as mixtures of two or more kinds. The organic liquid component can be appropriately determined by other factors of the patch, and fatty acid ester and more preferably glycerin fatty acid ester are suitably used from the standpoint of compatibility with the adhesive.

The fatty acid constituting fatty acid ester includes a saturated fatty acid such as caprylic acid, capric acid and 2-ethyhexynoic acid. Those can be used alone or two or more kinds can be used. Those saturated fatty acids do not have an unsaturated bond and therefore can prevent oxidative deterioration of the adhesive, that is, the adhesive layer.

In the case that the above-described caprylic acid or the like has been used as the saturated fatty acid, glyceryl tricaprylate, glyceryl tricaprate and glyceryl tri-2-ethylhexanoate are obtained as esters of various saturated fatty acids and an alcohol. Of those fatty acid esters, glyceryl tricaprylate as one kind of glycerin fatty acid ester is more preferably used from the point of compatibility with the adhesive.

The content of the organic liquid component in the first adhesive layer is that the upper limit is preferably 60 mass % and more preferably 50 mass % and the lower limit is preferably 5 mass %, more preferably 10 mass %, still more preferably 15 mass % and particularly preferably 20 mass %. The content ratio of the organic liquid component per 100 parts by mass of the adhesive is that the upper limit is preferably 200 parts by mass, more preferably 100 parts by mass, still more preferably 80 parts by mass and particularly preferably 70 parts by mass and the lower limit is preferably 10 parts by mass, more preferably 15 parts by mass, still more preferably 20 parts by mass and particularly preferably 30 parts by mass. When the content and content ratio of the organic liquid component are in the above ranges, sufficient flexibility can be imparted to the first adhesive layer. As a result, the patch has good stickiness to the movement of a skin surface and additionally can suppress physical skin irritation when peeling the patch from a skin. Furthermore, the organic liquid component contained in the first adhesive layer ooze to a back side (second surface-side) of the polyurethane film as a support. As a result, it is assumed that this contributes to achieve conflicting properties of peelability and adhesiveness of the cover tape to the polyurethane film even though a release treatment such as a silicone resin treatment is not applied to the back of the polyurethane film.

Crosslinking treatment may be applied to the first adhesive layer in order to impart appropriate cohesive force thereto. The crosslinking treatment includes physical crosslinking by irradiation of ionizing radiation such as electron beams, γ ray or X rays and chemical crosslinking by a crosslinking agent. Chemical crosslinking treatment by a crosslinking agent is preferably applied from the standpoint that crosslinking is performed with good handleability and reproducibility. For example, a crosslinking treatment is performed by adding a crosslinking agent to an adhesive composition having added thereto materials constituting the first adhesive layer, and conducting a heat treatment. As the crosslinking agent, a crosslinking agent used in performing a crosslinking treatment, such as an isocyanate crosslinking agent, a peroxide crosslinking agent or a metal chelate crosslinking agent, can be used.

The first adhesive layer to which crosslinking treatment has been applied as above holds appropriate balance between stickiness and cohesive properties by the degree of crosslinking, but the molecular weight and molecular weight distribution of the adhesive also give influence to those adjustments.

The thickness of the first adhesive layer is not particularly limited and can be appropriately set depending on a use of the patch of the present invention, a region to which the patch is stuck, and the like. As one example, the upper limit of the thickness is preferably 100 µm, more preferably 50 µm and still more preferably 35 µm, and the lower limit is preferably 10 µm, more preferably 16 µm and still more preferably 20 µm. When the thickness of the first adhesive layer is in the above range, appropriate adhesive force to a skin surface can be imparted.

In the patch of the present invention, the cover tape has a base material and a second adhesive layer. The base material used in the cover tape specifically includes plastic films such as a polyolefin film (polyethylene film, polypropylene film or the like) and a polyester film (polyethylene terephthalate film or the like). The thickness of the base material is that the upper limit is preferably 80 µm and more preferably 70 µm and the lower limit is preferably 30 µm and more preferably 40 µm.

The adhesive used in the second adhesive layer is not particularly limited, and adhesives generally used are used. The same adhesives as used in the first adhesive layer can be used. Above all, an acrylic adhesive can be suitably used from the standpoints of adhesiveness and peelability to the polyurethane film as a support. The thickness of the second adhesive layer is preferably 5 to 30 µm and more preferably 5 to 15 µm.

In the patch of the present invention, to prevent contamination of the surface of the first adhesive layer, the surface of the first adhesive layer is covered with a release liner until use as necessary. The release liner can use a release liner used in general adhesive tapes. A base material for the release liner specifically includes plastic films such as a polyethylene terephthalate film, a polyimide film, a polypropylene film, a polyethylene film, a polycarbonate film and a polyester film; metal-deposited plastic films of those films having a metal deposited thereon; papers such as Japanese paper, western paper, craft paper, glassine paper and wood-free paper; base materials formed by a fibrous material such as nonwoven fabric or cloth; and metal foils. The above base materials for the release liner, the surface of which being coated with a release agent having peeling performance, such as a silicone resin or a fluorine resin, can be used as the release liner.

FIG. 1 shows a schematic cross-sectional view of the patch according to one embodiment of the present invention. A patch 10 of the present embodiment is a patch of the embodiment having a printed layer 7 and a release liner 5. In the patch 10, a first adhesive layer 2 is provided on one surface (first surface) of a polyurethane film 1, and an adhesive surface of the first adhesive layer 2 is covered with the release liner 5. The printed layer 7 is formed on other surface (second surface) of the polyurethane film 1, and a cover tape 6 comprising a base material 3 and a second adhesive layer 4 is laminated on the printed layer 7 such that an adhesive surface side of the second adhesive layer 4 faces the other surface (second surface) of the polyurethane film 1.

A method of sticking the patch of the present invention to a skin surface is exemplified below. The release liner is peeled from the patch to expose the first adhesive layer, and a first adhesive layer-side of the patch is stuck to a skin surface and held by hand from the upper of the patch so as to be closely contacted with the skin surface. Thereafter, the cover tape is slowly peeled and sticking to a skin surface is completed. By the sticking method, the patch having high flexibility can be neatly and easily stuck to a skin surface without wrinkles.

In order to easily peel the release liner in the state that the cover tape of the patch of the present invention is closely contacted with the polyurethane film, peel force between the first adhesive layer and the release liner is preferably smaller than the peel force between the second adhesive layer and the polyurethane film (second surface-side of the polyurethane film). The peel force between the second adhesive layer and the polyurethane film is that the upper limit is preferably 0.60 N/20 mm width and more preferably 0.50 N/20 mm width and the lower limit is preferably 0.15 N/20 mm width and more preferably 0.20 N/20 mm width. When the peel force between the second adhesive layer and the polyurethane film is less than 0.15 N/20 mm width, there is a possibility that the cover tape is peeled during storage of the patch or the cover tape is peeled prior to peeling of the release liner when using the patch. On the other hand, when the peel force between the second adhesive layer and the polyurethane film exceeds 0.60 N/20 mm width, peeling of the cover tape is heavy, and there is a possibility that when the cover tape is peeled after sticking the patch to a skin, the patch is peeled together with the cover tape.

### Examples

The present invention is further specifically described below by reference to examples, but those examples do not limit the present invention. The terms "parts" and "%" used hereinafter mean "parts by mass" and "mass %", respectively. In the present description, the term "parts by mass" is substantially synonymous with "parts by weight" and the term "mass %" is substantially synonymous with "weight %".

### (Comparative Example 1)

A monomer mixture comprising 65 parts of isononyl acrylate, 30 parts of 2-methoxyethyl acrylate and 5 parts of acrylic acid were subjected to a copolymerization reaction in toluene to obtain a toluene solution of an acrylic adhesive. 60 Parts of glyceryl tricaprylate as an organic liquid component and 0.04 parts of a trifunctional isocyanate compound as a crosslinking agent, each per 100 parts of the acrylic adhesive, were added to the solution of the acrylic adhesive obtained to prepare a uniform adhesive solution.

The adhesive solution was then applied to a release-treated surface of a paper release liner (thickness: 97 µm), one surface of which having been subjected to a release treatment by a silicone resin, such that the thickness after drying was 25 µm, and then dried. Thus, an adhesive layer was formed. The adhesive layer was adhered to a polyurethane film surface of a laminate film comprising the polyurethane film having a thickness of 8 µm and a protective film (polypropylene film having a thickness of 40 µm). Thus, a patch of Comparative Example 1 was obtained.

### (Example 1)

After peeling the polypropylene film (protective film) of the patch obtained in Comparative Example 1, an adhesive layer-side of a cover tape (product name: SPV-362MK, manufactured by Nitto Denko Corporation) comprising a base material and the adhesive layer was then adhered to a polyurethane film surface of the laminated film. Thus, a patch of Example 1 was obtained.

### (Example 2)

After peeling the polypropylene film (protective film) of the patch obtained in Comparative Example 1, an ink was then applied to a polyurethane film surface using a laser printer to form a printed layer. An adhesive layer-side of a cover tape (product name: SPV-362MK, manufactured by Nitto Denko Corporation) comprising a base material and the adhesive layer was adhered to the printed layer. Thus, a patch of Example 2 was obtained.

### (Example 3)

A patch of Example 3 was obtained in the same manner as in Example 1, except that a polyurethane film having a thickness of 30 µm was used in place of the polyurethane film having a thickness of 8 µm.

### (Comparative Example 2)

A patch of Comparative Example 2 was obtained in the same manner as in Example 1, except that a polyethylene terephthalate film having a thickness of 12 µm was used in place of the polyurethane film having a thickness of 8 µm.

### (Comparative Example 3)

The acrylic adhesive solution (not containing an organic liquid component and a crosslinking agent) in Comparative Example 1 was applied to a release-treated surface of a paper release liner (thickness: 97 µm), one surface of which having been subjected to a release treatment by a silicone resin, such that the thickness after drying was 25 µm, and then dried. Thus, an adhesive layer was formed. The adhesive layer was adhered to a polyurethane film surface of a laminate film comprising the polyurethane film having a thickness of 8 µm and a polypropylene film (protective film).

After peeling the polypropylene film (protective film), an adhesive layer-side of a cover tape (product name: SPV-362MK, manufactured by Nitto Denko Corporation) comprising a base material and the adhesive layer was then adhered to a polyurethane film surface. Thus, a patch of Comparative Example 3 was obtained.

### (Peel force)

The patches of Examples 1 to 3 and Comparative Examples 1 to 3 each were cut into a ribbon shape having a width of 20 mm and a length of 100 mm to prepare a test piece. After peeling the release liner of the test piece, an adhesive layer surface was adhered to a phenol resin laminate plate by reciprocating one time a 2 kg rubber roller in a rate of 50 mm/sec. In one end in a longitudinal direction of the test piece, about 5 mm was peeled from the edge of the cover tape (protective film in Comparative Example 1) and turned up at an angle of 180°, and an auxiliary paper (width: about 20 mm, and length: about 200 mm) was adhered thereto to extend the length. Using a tensile tester, one end of the auxiliary paper was sandwiched between upper hooks, the phenol resin laminate plate was sandwiched between lower hooks, the cover tape (protective film in Comparative Example 1) was peeled successively in 180° direction in a rate of 300 mm/min in an atmosphere of 23°C±2°C and 50±10% RH, and peel force was measured (average value of n=3).

### (Workability)

The patches of Examples 1 to 3 and Comparative Examples 1 to 3 each were cut into a substantially square shape of one side 30 mm × 30mm to prepare a test piece. The release liner of the test piece was peeled, and an adhesive layer surface was stuck to an arm of a trialist. After holding the test piece by hand for 10 seconds, the state of the test piece when the edge of the cover tape (protective film in Comparative Example 1) was pinched with fingers and peeled was evaluated on the basis of the following criteria, and evaluation points were calculated (average value of n=3).
5: Test piece does not float from arm and does not wrinkle
3. Test piece slightly floats from arm but does not wrinkle
1. Test piece floats from arm and wrinkles

### (Re-stickiness)

The patches of Examples 1 to 3 and Comparative Examples 1 to 3 each were cut into a substantially square shape of one side 30 mm × 30mm to prepare a test piece. After peeling the release liner of the test piece, an adhesive layer surface was adhered to a phenol resin laminate plate by reciprocating one time a 2 kg rubber roller in a rate of 50 mm/sec. The edge of the cover tape (protective film in Comparative Example 1) was pinched with fingers and partially peeled. The state of the test piece when the peeled part was again stuck was evaluated by the following evaluations criteria, and evaluation points were calculated (average value of n=3).
3: Peeled part was stuck
1: Peeled part was not stuck

**[Table 1]**

| | Peel force (N/20 mm width) | Workability | Re-stickiness |
|---|---|---|---|
| Example 1 | 0.21 | 5.0 | 3.0 |
| Example 2 | 0.52 | 5.0 | 3.0 |
| Example 3 | 0.29 | 5.0 | 3.0 |
| Comparative Example 1 | 0.10 | 5.0 | 1.0 |
| Comparative Example 2 | 1.74 | 4.3 | 3.0 |
| Comparative Example 3 | 0.71 | 1.0 | 3.0 |

The results are shown in Table 1.

The patches of Examples 1 to 3 were that even though the edge of the cover tape was peeled during storage, the peeled edge could be again stuck to the polyurethane film, and additionally the cover tape could be easily peeled when using. On the other hand, the patch of Comparative Example 1 was that when the protective film was peeled during storage, the peeled protective film could not be again stuck. Furthermore, the patches of Comparative Examples 2 and 3 were that when the cover tape was peeled after sticking to a skin surface, the patches floated from a skin surface and was difficult to stick neatly and easily.

Although the present invention has been described in detail and by reference to the specific embodiments, it is apparent to one skilled in the art that various modifications or changes can be made without departing the spirit and scope of the present invention.

This application is based on Japanese Patent Application No. 2018-091878 filed May 11, 2018, the disclosure of which is incorporated herein by reference.

### REFERENCE SIGNS LIST

1: Polyurethane film
2: First adhesive layer
3: Base material
4: Second adhesive layer
5: Release liner
6: Cover tape
7: Printed layer
10: Patch

## Claims

1. A patch including a polyurethane film, a first adhesive layer provided on one surface of the polyurethane film, and a cover tape provided on the other surface of the polyurethane film,
wherein the cover tape includes a base material and a second adhesive layer provided on one surface of the base material and an adhesive surface of the second adhesive layer is laminated on the other surface-side of the polyurethane film, and
the first adhesive layer contains an adhesive and an organic liquid component.

2. The patch according to claim 1, wherein a printed layer is formed on at least a part of the other surface of the polyurethane film.

3. The patch described according to claim 1 or 2, wherein a content of the organic liquid component in the first adhesive layer is 10 to 60 mass %.

4. The patch according to any one of claims 1 to 3, wherein the organic liquid component contains a fatty acid ester.

5. The patch according to any one of claims 1 to 4, wherein the adhesive contains an acrylic adhesive.
